Europäisches Patentamt

**European Patent Office**

**Office européen des brevets**

(19)

(11) Numéro de publication: **0 079 284**
**B1**

(12) **FASCICULE DE BREVET EUROPÉEN**

(45) Date de publication du fascicule du brevet:
24.09.86

(51) Int. Cl.⁴: **A 61 B 10/00**

(21) Numéro de dépôt: **82402042.4**

(22) Date de dépôt: **05.11.82**

(54) Sonde ultrasonore à balayage mécanique sectoriel.

(30) Priorité: **06.11.81 FR 8120861**

(43) Date de publication de la demande:
**18.05.83 Bulletin 83/20**

(45) Mention de la délivrance du brevet:
**24.09.86 Bulletin 86/39**

(84) Etats contractants désignés:
**DE GB**

(56) Documents cité:
**EP-A-0 045 265**
**DE-A-1 466 912**
**DE-A-2 924 194**
**FR-A-2 298 107**
**FR-A-2 461 486**
**GB-A-2 067 759**
**US-A-3 741 004**
**US-A-4 215 585**

(73) Titulaire: **CGR ULTRASONIC, 9, Chaussée de Paris,
F-77102 Villenoy- les- Meaux (FR)**

(72) Inventeur: **Prud'hon, Lucien, THOMSON- CSF SCPI
173, bld Haussmann, F-75379 Paris Cedex 08 (FR)**

(74) Mandataire: **Grynwald, Albert, THOMSON- CSF
SCPI 19, avenue de Messine, F-75008 Paris (FR)**

LIBER, STOCKHOLM 1986

## Description

La présente invention concerne une sonde ultrasonore à balayage mécanique sectoriel. Elle est particulièrement adaptée à l'examen échographique médical. En particulier elle trouve application dans les examens réclamant de petites fenêtres d'accès.

En examen ultrasonore, il est connu d'utiliser des sondes sectorielles pour émettre et/ou recevoir les ondes ultrasonores. Ces sondes comportent un élément piézo-électrique, source et récepteur des ondes ultrasonores. Un avantage particulier de certaines de ces sondes est de fournir un faisceau d'ultrasons, fin et bien directif, qui se répète indépendamment de l'angle qu'il occupe dans le secteur. L'élément piézo-électrique est mû mécaniquement dans un boitier couplé acoustiquement du corps en examen. D'autres sondes sectorielles, qui utilisent des propriétés des ondes déphasées selon une analogie bien connue avec les antennes radar, n'ont pas cet avantage. Par contre, elles évitent les problèmes mécaniques. En effet, l'usage d'un moteur pour réaliser mécaniqment le changement d'orientation du faisceau entraîne des problèmes de jeux mécaniques et de précision dans le repérage de la position angulaire du faisceau.

Un secteur de balayage est défini dans le plan d'examen. Dans ce plan, entre deux droites sécantes à l'intersection desquelles est placée la sonde, un faisceau ultrasonore balaye le domaine limité par les deux droites. Le faisceau admet un axe central qui prend donc, dans la durée du balayage du secteur, une succession de positions instantannées dans le secteur. Le secteur admet un axe central qui peut être par exemple la bissectrice des deux droites qui le limitent. L'ouverture du secteur est mesurée par l'angle séparant les deux droites limites.

Dans un cas particulier, fréquemment rencontré, le faisceau ultrasonore n'est pas permanent pendant le balayage du secteur. Dans ce cas, la sonde travaille alternativement en émission et en réception. A l'émission, la sonde est électriquement connectée à un générateur d'impulsions qui excitent la source d'ultrasons. Cette sonde comporte un transducteur piézo-électrique qui transforme un signal électrique en un signal acoustique ici ultrasonique, puis la sonde est connectée sur un circuit récepteur des échos ultrasonores renvoyés par les obstacles rencontrés sur le trajet du faisceau. L'effet piézo-électrique étant réversible, le transducteur convertit les échos ultrasonores en signal électrique représentatif des obstacles rencontrés. On obtient ainsi une information de la nature des obstacles rencontrés dans une direction d'émission. Puis la sonde est à nouveau commutée sur son génerateur d'impulsions et tournée dans une autre direction. On appelle fréquence de répétition le nombre de directions le long desquelles un faisceau ultrasonore est émis et les échos reçus pendant une unité de temps.

On connaît un type de sonde mécanique comportant un transducteur monté pivotant par rapport à un axe et actionnée au moyen d'une came tournant dans un plan perpendculaire à la direction moyenne d'émission et entraînée en rotation par un moteur électrique, le contact entre la came et l'équipage pivotant étant assuré par un ressort le sollicitant en permanence vers la came. Un tel dispositif est par exemple décrit dans le brevet US-4 215 585. L'invention concerne un type de sonde plus simple et plus fiable présentant notamment la particularité de ne nécessiter aucun ressort (par nature fragile et aux caractéristiques variables dans le temps), tout en conservant les qualités essentielles d'un tel type de sonde.

La présente invention permet d'améliorer les caractéristiques de sondes mécaniques. En particulier, elle permet la réalisation d'une sonde de très petites dimensions. Cette caractéristique permet de placer la sonde sur des fenêtres anatomiques étroites. En effet, les ultra-sons sont arrêtés par les os et absorbés par les poches d'air. La construction d'une image doit donc être faite sur des fenêtres anatomiques ne comportant, ni poches d'air, ni os.

C'est un autre avantage de la présente invention de fournir une sonde dont l'orientation angulaire est directement liée à un mouvement circulaire uniforme. Cette caractéristique permet d'alléger les moyens de repérage angulaire de la position instantannée de l'axe central du faisceau ultrasonore.

C'est un autre avantage selon l'invention que de permettre la construction d'une sonde qu'il est possible d'arrêter sur un angle de déviation quelconque à l'intérieur du secteur de balayage. Dans une telle application, il est possible ainsi d'arrêter l'examen sur n'importe quelle position du secteur.

Un autre avantage selon l'invention consiste en la possibilité de montage de transducteurs piézo-électriques de fréquences propres différentes Il est possible ainsi de monter et de démonter tout type de transducteur piézo-électrique Un autre avantage selon l'invention est de permettre des balayages sectoriels d'angle quelconque le secteur pouvant avoir une ouverture comprise de quelques degrés autour de la normale, a 90 de part et d'autre de cette normale. La frequence de répétition peut être de l'ordre de 25 a 40 hertz

La sonde ultrasonore selon l'invention du type à balayage sectoriel mécanique, comportant un équipage mobile, muni d'un élément transducteur, monté pivotant par rapport a un axe, un moyen moteur et des moyens de transmission de mouvement dudit moyen moteur audit équipage mobile, lesdits moyens de transmission comportant une came annulaire dont une piste de guidage coopère avec un élément de transmission, est caractérisée en ce que la came annulaire est liée à l'équipage mobile et comporte en profil évolutif selon la direction d'émission du transducteur, et l'élément de transmission est lié au moyen moteur.

D'autres éléments de l'invention seront expliqués à l'aide de la description et des figures qui sont:

la figure 1: un schéma général de sondes selon l'invention,

la figure 2: un schéma d'un premier mode de réalisation d'une sonde selon l'invention,

la figure 3: une variante du premier mode de réalisation selon l'invention,

la figure 4: un autre mode de réalisation selon l'invention,

la figure 5: une variante du mode de réalisation de la figure 4,

la figure 6: quatre diagrammes d'évolution des angles et des vitesses de deux sondes selon l'invention.

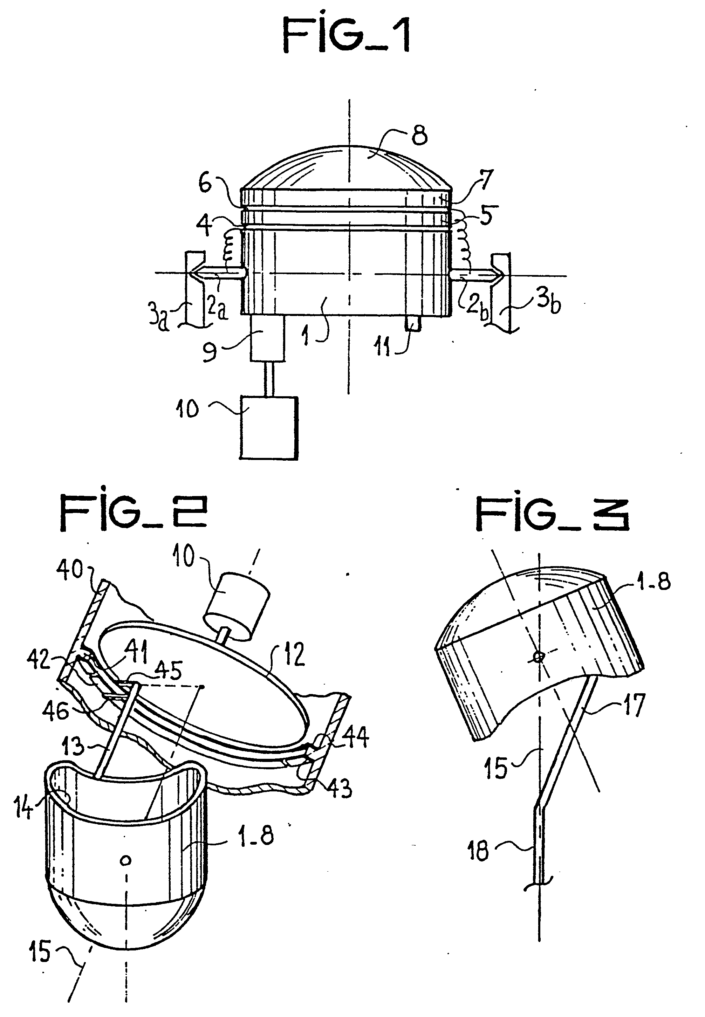

A la figure 1, est représentée une sonde selon l'invention. Elle comporte un élément transducteur 5 collé sur un amortisseur 1. L'amortisseur 1 a pour rôle.d'absorber le rayonnement arrière de l'élément traducteur. L'élément transducteur 5 porte aussi une lame d'adaptation quart d'onde 7, et une lentille acoustique 8 destinée a focaliser l'énergie ultrasonore. Sur une droite passant par le barycentre de l'ensemble mobile 1-8 de la sonde, deux pivots conducteurs alignés 2a, 2b sont engagés dans deux supports conducteurs 3a et 3b respectifs. Ces supports conducteurs ont pour premier rôle de permettre un pivotement autour de l'axe passant par le barycentre de l'ensemble mobile de la sonde et pour second rôle d'assurer le transfert des signaux électriques à l'émission et à la réception entre un connecteur non représenté et le transducteur 5. Des électrodes 4 et 6 recouvrent les deux faces opposées du transducteur et sont reliées par fils aux pivots 2a et 2b, respectivement. Les connexions peuvent être constituées par des liaisons en spirale entre les pivots 2 et les supports conducteurs 3. Le pivot peut être posé sur un roulement à billes, non représenté, placé sur le support conducteur 3. A l'arrière de l'amortisseur 1, est placé un moyen 9 de transformation du mouvement rotatif permanent du moteur 10 en un mouvement oscillatoire alternatif de l'ensemble mobile de la sonde sur l'axe des pivots. Un codeur de positions angulaires 11 est placé sur l'arrière de l'amortisseur 1. Il est possible de réaliser d'une manière classique un asservissement en vitesse du moteur 10 avec le codeur de position 11.

A la figure 2 est représenté un premier mode de réalisation de sonde selon l'invention. Le moteur 10 porte un axe sur lequel est fixé un disque 12. L'axe 15 du moteur et du disque 12 est aussi l'axe central du secteur balayé par la sonde. Cet axe central 15 passe donc par le barycentre de l'ensemble mobile 1-8 de la sonde. Un levier 13 est fixé à la périphérie du disque 12 animé d'un mouvement de rotation continue. L'amortisseur arrière 1 de la sonde porte un profil de came en cloche 14. Les moyens de liaison de l'extrémité libre du levier 13 avec la came 14 peuvent être de natures différentes. Dans le cas d'une liaison mécanique, le levier 13 a son extrémité libre engagée dans une rainure-glissière (non représentée) de la came-cloche 14 de façon à ce qu'elle reste constamment liée au profil de la came. Afin d'améliorer le glissement du levier 13 dans la rainure de la came 14, il est possible de fixer des moyens de roulement sur l'extrémité du levier 13. Ces moyens de roulement non representés peuvent comporter un agent lubrifiant et/ou un roulement genre roulement à bille ou à aiguille. Dans un autre mode d'exécution l'extrémité du levier 13 porte un aimant ou un electroaimant qui exerce une action permanente et durable sur le bord de la came 14.

Le bord de la came 14 porte une partie active magnétique qui peut être ou bien un pôle magnétique d'une polarité opposée à celle de l'aimant porté par le levier 13, ou bien une piste en un matériau magnétique comme du permalloy ou un acier au silicium. Quand le levier 13 porte un électro-aimant, il faut des moyens de connexion électrique sur le levier pour le courant de l'électro-aimant. Les contacts qui prélèvent ce courant peuvent être du type à contact tournant sur piste circulaire statique. Un balai 45, 46 sur chaque piste établit un contact permanent sur la piste portée de l'extérieur de la sonde à un potentiel convenable par le connecteur de sonde. Chaque balai est mécaniquement lié au levier qui l'entraine en rotation.

Le profil, calculé d'une manière connue, de la came 14 permet que le secteur d'observation soit balayé entre deux droites limites avec une vitesse de balayage qui dépend de la vitesse de rotation du moteur mais aussi de la courbure du profil de la came. Il est ainsi possible par exemple en réalisant des profils de cames comportant un plateau au milieu du secteur de ralentir le balayage du secteur et donc de permettre d'augmenter le nombre de faisceaux d'exploration localement dans le secteur sans toucher à la fréquence de répétition.

L'intérêt d'un profil calculé de came 14 réside dans la possibilité de permettre d'éviter les pertes de contrôle de l'oscillation de l'équipage mobile. En effet, dans un mouvement oscillatoire circulaire, un point du solide oscillant, hors de l'axe de rotation, décrit un arc de cercle. Aux extrémités de l'arc de cercle, la vitesse du point s'annule mais son accélération peut être très elevée. L'inertie de rotation fait que, pendant une fraction de temps, l'équipage mobile n'est plus controlé si on ne tient pas compte des inerties.

D'autre part, le fait de rendre la vitesse de rotation aussi constante que possible permet d'obtenir un repérage angulaire aisé d'une part, et que la sonde puisse émettre en des positions angulaires fixes. C'est un but de la présente invention de permettre un repérage facile sans nécessité absolue de réaliser une boucle d'asservissement en vitesse du moteur.

En effet, dans les dispositifs de l'art antérieur, il est souvent placé un codeur de position instantannée sur la sonde. Un comparateur du signal de position à une forme d'oscillation prédéterminée fournit une indication a un circuit de commande de l'alimentation du moteur électrique de la sonde. Ces dispositifs qui doivent être très rapides sont coûteux, augmentent la taille de la sonde et la dissipation thermique. Ces trois inconvénients sont éliminés dans la présente invention.

En effet, le profil de la came est calculé de telle sorte que la vitesse de rotation instantannée de l'équipage mobile soit constante pendant un sens de parcours de l'oscillation. Quand le sens s'inverse, la vitesse change rapidement et continûment de signe et reprend la même valeur absolue. De plus, de période en période, les caractéristiques cinématiques de la sonde se répétent indéfiniment.

Une autre supériorité du dispositif selon l'invention est de permettre que pendant le changement de sens de l'oscillation, l'equipage mobile 1-8 est conservé sous contrôle du moteur puisque la liaison came 14 - levier 13 est constante.

Dans le cas où la liaison came 14 - levier 13 est du type magnétique, qui a l'avantage de limiter les frottements et de permettre un ajustage plus grossier du profil de la came 14, un effet supplémentaire peut être offert. Quand le champ magnétique de liaison est fourni par un électro-aimant, l'accélération du mouvement d'oscillation peut être controlé magnétiquement. Quand l'angle dorientation du transducteur est proche d'une des valeurs extrêmes correspondantes aux droites limites du secteur balayé, l'électro-aimant peut fournir une énergie pour contrebalancer l'énergie d'inertie. Autrement dit, il faut fournir à l'équipage mobile une force de rappel qui tend à s'opposer à la force d'inertie. Il faut augmenter localement l'accélération au changement du sens d'oscillation.

A la figure 6, on a représenté quatre graphiques. Le graphique 6-1 représente l'évolution de l'orientation angulaire α du traducteur dans un cas idéal. Le graphique 6-2 correspond à la vitesse angulaire

$$\frac{d\alpha}{dt}$$

en fonction du temps. Les graphiques 6-3 et 6-4 représentent les mêmes grandeurs dans un cas réel de l'invention sans correction magnétique. Le but de la correction magnétique est de rapprocher la réponse de la sonde réelle du cas idéal.

Aux diagrammes 6-1 et 6-2, il est clair que la loi α (t) est parfaitement linéaire. Le changement de sens d'oscillation se fait aux demis périodes d'oscillations k.T/2 successives. Le repérage angulaire est précis et n'a pas besoin de capteur de position ni d'asservissement du moteur 10 en vitesse.

Au diagrammes 6-3 et 6-4, on constate que cette situation n'est vraie réellement qu'en dehors des changements de sens d'oscillation. Ainsi à l'instant

$$\frac{T}{2} - \left( \frac{\tau}{?} \right)$$

l'angle α n'évolue plus linéairement, la vitesse

$$\frac{d\alpha}{dt}$$

décroit et s'annule à l'instant

$$\frac{T}{2}$$

puis reprend sa valeur nominale à

$$\frac{T}{2} + \tau$$

en ayant changé de signe. A chaque changement de sens, pendant une durée de 2 $\tau$, la sonde est parfaitement controlée grâce à la came, mais il est impossible d'émettre des faisceaux ultrasonores dans cette partie du secteur d'examen. Pour améliorer cette situation, il faut augmenter la pente de la courbe

$$\frac{d\alpha}{dt}$$

à t =

$$\frac{kT}{2}$$

c'est à dire à chaque changement de sens d'oscillation. Autrement dit, il faut diminuer l'accélération de l'équipage mobile 1-8 quand la vitesse décroit et l'augmenter quand la vitesse croit.

Une solution à ce problème consiste à fournir une force éléctromagnétique pendant la durée de 2 $\tau$ (valant environ 10μs) sur l'équipage mobile. Pour celà, dans un premier mode d'exécution, sur la piste amenée du courant à l'électro-aimant porte par le levier 13, il est prévu une connexion spéciale à un courant 1 de valeur supérieure pour augmenter la force électromagnétique pendant la durée 2τ et ainsi contrebalancer par attraction magnétique l'inertie mécanique de l'équipage mobile 1-8.

A la figure 2, on a représenté ce mode de réalisation. Sur cette figure, le boitier 40 est vu partiellement et en coupe sur une demi-circonférence. Le support de piste 41 est un anneau isolant et acoustiquement neutre qui porte sur sa face supérieure une piste conductrice 43 qui est portée à un potentiel positif par l'intermédiaire du connecteur de sonde non représenté. La face inférieure du support 41 porte une piste conductrice 44 portée à un potentiel négatif pour le retour du courant de l'électro-aimant. Un secteur de surcourant 42 permet localement au passage de l'équipage mobile 1-8 sur le renversement de sens d'oscillation d'exercer une force d'attraction supplémentaire du levier 13 sur la came 14.

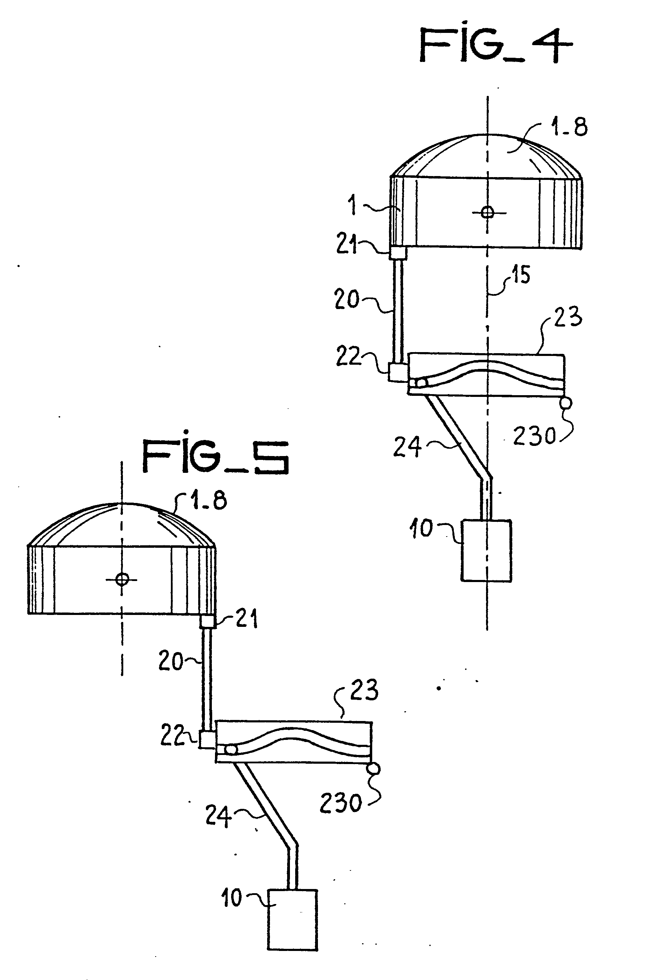

A la figure 3 est représentée une variante de ce montage. L'axe du moteur 10 comporte un levier coudé 17 qui décrit en permanance un cercle autour de l'axe 18 du moteur. L'axe central du faisceau est confondu avec l'axe 18 du moteur.

A la figure 4 est représenté un autre mode de réalisation selon l'invention. L'amortisseur 1 porte sur sa face arrière en un point oscillant, une articulation 21 reliée a une articulation 22 par une biellette 20. L'articulation 22 est montée à l'extrêmite diamétrale d'une came 23 fixée sur une articulation 230 elle même fixe par rapport à l'équipage mobile. La came 23 est en forme d'anneau de cylindre creux et porte sur sa face intérieure une rainure dont la forme est calculée selon le principe de la came 14 des figures 2 ou 3. Cet anneau est une came mobile qui permet d'équilibrer le moment d'inertie de l'équipage mobile 1-8. Un levier coudé 24, comme il a été décrit figure 3, est fixé à l'axe central du moteur 10. L'axe coudé 24 comporte à son extrémité supérieure une partie horizontale qui reste constamment à l'intérieur de la fente rectiligne pratiquée dans la came 23. Le mouvement permanent de rotation du moteur est donc transformé par l'intermédiaire de la came 23 et du levier 20 en un mouvement alternatif d'oscillation autour des pivots pour l'équipage mobile.

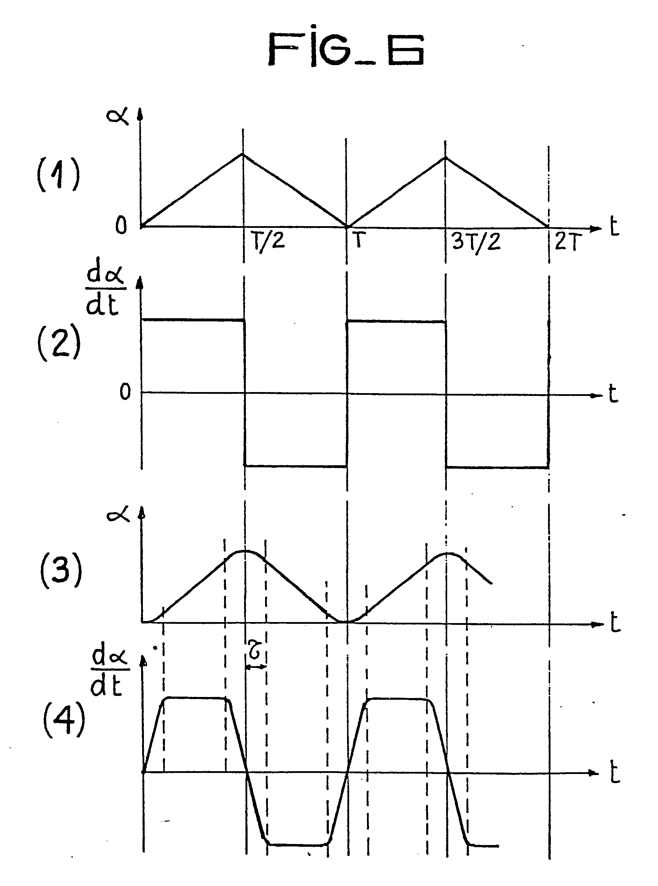

A la figure 5, une variante de la figure 4 est présentée. L'équipage mobile 1-8 a été tourné de 180° dans le plan de la figure de façon à ce que les mouvements de l'équipage mobile 1-8 et de la came 23 soient toujours en opposition. Cette disposition permet d'équilibrer les moments des couples en particulier aux changements de sens des oscillations. On évite ainsi une secousse sur l'équipage mobile à chaque oscillation.

D'autre réalisations de la présente invention sont aussi protégées. En particulier, il est possible d'utiliser la compensation magnétique aussi dans les sondes décrites figures 4 et 5.

## Revendications

1. Sonde ultrasonore à balayage sectoriel mécanique comportant un équipage mobile (1-8), muni d'un élément transducteur (5), monté pivotant par rapport à un axe (2a, 2b), un moyen moteur (10) et des moyens de transmission de mouvement dudit moyen moteur audit équipage mobile, lesdits moyens de transmission comportant une came annulaire (14, 23) dont une piste de guidage coopère avec un élément de transmission (13, 17, 24), caractérisée en ce que la came annulaire est liée à l'équipage mobile et comporte un profil évolutif selon la direction d'émission du transducteur, et l'élément de transmission est lié au moyen moteur.

2. Sonde ultrasonore selon la revedication 1, caractérisée en ce que ladite came (14) est définie sur l'équipage mobile lui-même.

3. Sonde ultrasonore selon la revendication 2, caractérisée en ce que ladite came est définie sur un élément amortisseur (1) dudit équipage mobile.

4. Sonde ultrasonore selon la revendication 1, caractérisée en ce que ladite came (23) est articulée en un point de sa périphérie et reliée audit équipage mobile par une biellette (20) articulée à ses deux extrémités.

5. Sonde selon la revendication 2 ou 3, caractérisée en ce que la came (14) est balayée par un levier (13) qui porte des moyens de liaison en contact avec la came (14), le levier (13) étant porté par une roue (12) entraînée en rotation permanente par le moteur (10).

6. Sonde selon la revendication 2 ou 3, caractérisée en ce que le moteur (10) porte un axe (18) confondu avec l'axe central du secteur balayé et prolongé par un levier coudé (17) dont l'extrêmité porte des moyens de liaison avec la came (14).

7. Sonde selon la revendication 4, caractérisée en ce qu'elle comporte un levier coudé (24) entraîné par ledit moyen moteur et dont l'extrémité libre est munie de moyens de liaison avec une came constituée d'une rainure pratiquée sur la face intérieure d'un anneau cylindrique (23) articulé sur un axe (230), fixé à un diamètre de l'anneau (23), le profil de la rainure étant déterminé de manière à transformer le mouvement de rotation permanent du moteur (10) en un mouvement oscillatoire transmis au traducteur (5) par l'intermédiaire de ladite biellette.

8. Sonde selon la revendication 5 ou 6, caractérisée en ce que lesdits moyens de liaison comprennent des moyens de roulement disposés à l'extrêmité libre du levier (13 ou 17).

9. Sonde selon la revendication 8, caractérisée en ce que les moyens de roulement comportent un roulement à bille.

10. Sonde selon la revendication 2, caractérisée en ce que lesdits moyens de liaison sont de type magnétique.

11. Sonde selon la revendication 10, caractérisée en ce que l'extrémité du levier (13, 17, ou 24) porte un aimant qui coopère avec une partie magnétique active disposée sur la face en regard de la came (1 ou 23).

12. Sonde selon la revendication 11, caractérisée en ce que l'aimant porte par l'extrêmité libre du levier (13, 17 ou 24) est un électro-aimant.

13. Sonde selon la revendication 12, caractérisée par des moyens de connexion électrique dudit électroaimant comportant sur un support isolant de piste (41), une piste (43) portée à un potentiel donné et une piste (44) portée à un autre potentiel donné et des contacts glissants (45-46) fixés mécaniquement au levier, le support isolant de piste (41) étant porté par un boîtier (40).

14. Sonde selon la revendication 13, caractérisée en ce que localement, les moyens de connexion électriques comportent des moyens de commutation sur un secteur de surcourant.


**Patentansprüche**

1. Ultraschallsonde mit mechanischer sektorieller Ablenkung, welche eine bewegliche Einheit (1-8) aufweist, die mit einem Wandlerelement (5) versehen ist, das schwenkbar um eine Achse (2a, 2b) gelagert ist, mit einer Antriebseinrichtung (10) und Mitteln zur Übertragung der Bewegung der Antriebseinrichtung auf die genannte bewegliche Einheit, wobei diese Übertragungsmittel eine ringförmige Steuerkurve (14, 23) umfassen, deren Führungsbahn mit einem Übertragungselement (13, 17, 24) zusammenwirkt, dadurch gekennzeichnet, daß die ringförmige Steuerkurve mit der beweglichen Einheit verbunden ist und ein Profil aufweist, dessen Verlauf der Abstrahlrichtung des Wandlers entspricht, und das Übertragungselement an die Antriebseinrichtung angeschlossen ist.

2. Ultraschallsonde nach Anspruch 1, dadurch gekennzeichnet, daß die genannte Steuerkurve (14) an der beweglichen Einheit selbst gebildet ist.

3. Ultraschallsonde nach Anspruch 2, dadurch gekennzeichnet, daß die genannte Steuerkurve an einem Dämpfungselement (1) der genannten beweglichen Einheit gebildet ist.

4. Ultraschallsonde nach Anspruch 1, dadurch gekennzeichnet, daß die genannte Steuerkurve (23) an einem Punkte ihres Umfanges angelenkt und an die genannte bewegliche Einheit über ein Pleuel (20) angeschlossen ist, welches an seinen zwei Enden angelenkt ist.

5. Sonde nach Anspruch 2 oder 3, dadurch gekennzeichnet, daß die Steuerkurve (14) durch einen Hebel (13) abgetastet wird, welcher Mittel zur Kontaktverbindung mit der Steuerkurve (14) trägt, wobei der Hebel (13) durch ein Rad (12) getragen wird, das dauernd durch den Motor (10) in Drehung versetzt wird.

6. Sonde nach Anspruch 2 oder 3, dadurch gekennzeichnet, daß der Motor (10) eine Achse (18) trägt, welche mit der Mittelachse des überstrichenen Sektors zusammenfällt und durch einen gebogenen Hebel (17) verlängert ist, dessen Ende Mittel zur Verbindung mit der Steuerkurve (14) trägt.

7. Sonde nach Anspruch 4, dadurch gekennzeichnet, daß sie einen gebogenen Hebel (24) umfaßt, der durch die Antriebseinrichtung angetrieben wird und dessen freies Ende mit Mitteln zur Verbindung mit einer Steuerkurve versehen ist, welche durch eine Rille gebildet ist, die in der Innenfläche eines zylindrischen Ringes (23) gebildet ist, der auf einer Achse (230) angelenkt ist, die an einem Durchmesser des Ringes (23) befestigt ist, wobei das Profil der Rille derart bestimmt ist, daß die dauernde Drehbewegung des Motors (10) in eine Schwingbewegung umgesetzt wird, welche auf den Wandler (5) über das genannte Pleuel übertragen wird.

8. Sonde nach Anspruch 5 oder 6, dadurch gekennzeichnet, daß die genannten Verbindungsmittel Rollmittel umfassen, welche am freien Ende des Hebels (13 oder 17) angeordnet sind.

9. Sonde nach Anspruch 8, dadurch gekennzeichnet, daß die Rollmittel ein Kugellager umfassen.

10. Sonde nach Anspruch 2, dadurch gekennzeichnet, daß die genannten Verbindungsmittel von magnetischer Art sind.

11. Sonde nach Anspruch 10, dadurch gekennzeichnet, daß das Ende des Hebels (13, 17 oder 24) einen Magnet trägt, welcher mit einem magnetischen aktiven Teil zusammenwirkt, das auf der gegenüberliegenden Fläche der Steuerkurve (1 oder 23) angeordnet ist.

12. Sonde nach Anspruch 11, dadurch gekennzeichnet, daß der Magnet, den das freie Ende des Hebels (13, 17 oder 24) trägt, ein Elektromagnet ist.

13. Sonde nach Anspruch 12, gekennzeichnet durch Mittel zum elektrischen Anschließen des genannten Elektromagneten, welche auf einem isolierenden Bahnträger (41) eine Bahn (43) umfassen, die auf ein gegebenes Potential gelegt ist, und eine Bahn (44) umfaßt, welche auf ein anderes gegebenes Potential gelegt ist, sowie mechanisch fest mit dem Hebel verbundene Gleitkontakte (45-46) umfassen, wobei der isolierende Bahnträger (41) durch ein Gehäuse (40) getragen wird.

14. Sonde nach Anspruch 13, dadurch gekennzeichnet, daß die elektrischen Anschlußmittel Umschaltmittel zum Umschalten auf einen Überstromsektor umfassen.


**Claims**

1. Ultrasonic probe with mechanical sector scanning comprising a movable unit (1-8) equipped with a transducer element (5) mounted pivotally with respect to an axis (2a, 2b), a drive means (10) and means for transmitting the movement of the drive means to said movable unit, said transmission means comprising an annular cam (14, 23) whose guide track cooperates with a transmission element (13, 17, 24), characterized in that the annular cam is connected to the movable unit and comprises a profile evolved in accordance with the emission direction of the transducer and the transmission element is connected to the drive means.

2. Ultrasonic probe according to claim 1, characterized in that said cam (14) is defined on the movable unit itself.

3. Ultrasonic probe according to claim 2, characterized in that said cam is defined on a damping element (1) of said movable unit.

4. Ultrasonic probe according to claim 1, characterized in that said cam (23) is articulated at a point of its periphery and is connected to said movable unit by a link (20) articulated at its two ends.

5. Probe according to claim 2 or 3, characterized in that the cam (14) is scanned by a lever (13) which carries connecting means in contact with the cam (14), the lever (13) being carried by a wheel (12) permanently entrained in rotation by the motor (10).

6. Probe according to claim 2 or 3, characterized in that the motor (10) carries a shaft (18) which coincides with the centre axis of the scanned sector and is extended by a bent lever (17) whose end carries the means for connection to the cam (14).

7. Probe according to claim 4, characterized in that it comprises a bent lever (24) which is entrained by said drive means and the free end of which is equipped with means for connection to a cam formed by a groove formed in the interior face of a cylindrical ring (23) articulated on a shaft (230) fixed to a diameter of the ring (23), the profile of the groove being determined so as to transform the permanent rotational movement of the motor (10) into an oscillating movement transmitted to the transducer (5) via said link.

8. Probe according to claim 5 or 6, characterized in that said connecting means comprise rolling means disposed at the free end of the lever (13 or 17).

9. Probe according to claim 8, characterized in that the rolling means comprise a ball bearing.

10. Probe according to claim 2, characterized in that said connecting means are of magnetic type.

11. Probe according to claim 10, characterized in that the end of the lever (13, 17 or 24) carries a magnet which cooperates with a magnetic active part disposed on the face opposite the cam (1 or 23).

12. Probe according to claim 11, characterized in that the magnet carried by the free end of the lever (13, 17 or 24) is an electromagnet.

13. Probe according to claim 12, characterized by means for electrical connection of said electromagnet comprising an insulating track support (41), a track (43) brought to a given potential and a track (44) brought to another given potential, and sliding contacts (45 - 46) fixedly mechanically connected to the lever, the insulating track support (41) being carried by a housing (40).

14. Probe according to claim 13, characterized in that the electrical connection means comprise switchover means for switching over to an overcurrent sector.

0 079 284

FIG_1

FIG_2

FIG_3

FIG_4

FIG_5

# FIG_6